# EUROPEAN PATENT APPLICATION

(11) **EP 4 353 746 A1**
(43) Date of publication of application: **17.04.2024**
(21) Application number: 22819608.5
(22) Date of filing: 09.06.2022
(51) Int. Cl.: C07K 16/28, C12N 15/13, A61K 39/395, A61P 35/00

(54) **ANTI-PD-1 HUMANIZED ANTIBODY OR ANTIGEN-BINDING FRAGMENT THEREOF AND APPLICATION THEREOF**

(30) Priority: 11.06.2021 CN 202110654827
(71) Applicant: Guangdong Fapon Biopharma Inc., Dongguan, Guangdong 523808 (CN)
(72) Inventor: HUO, Yongting, Dongguan, Guangdong 523808 (CN); FU, Jun, Dongguan, Guangdong 523808 (CN); LU, Di, Dongguan, Guangdong 523808 (CN); ZHANG, Zhe, Dongguan, Guangdong 523808 (CN); LU, Lisheng, Dongguan, Guangdong 523808 (CN); LI, Fan, Dongguan, Guangdong 523808 (CN); GONG, Chunxi, Dongguan, Guangdong 523808 (CN)
(74) Representative: HGF
(86) International application number: PCT/CN2022/097854
(87) International publication number: WO 2022/258011

(57) **Abstract**

The present application relates to the field of biomedical technology, and specifically, provides an anti-PD-1 humanized antibody or an antigen-binding fragment thereof and an application thereof. The antibody can efficiently and specifically bind to PD-1, and can effectively block the binding of the PD-1 to PD-L1 and PD-L2. Therefore, the antibody or the antigen-binding fragment thereof, and its related nucleic acid, vector, cell or pharmaceutical composition can be used to prepare a medicament of PD-1-mediated diseases or conditions.

## Description

### Technical Field

The application belongs to the field of biomedical technology. More specifically, it relates to an anti-PD-1 humanized antibody or an antigen-binding fragment thereof and an application thereof.

### Background

A programmed death factor-1 (PD1) is a member of a CD28 family, and is expressed on activated B cells, T cells and myeloid cells. A human PD1 is encoded by a gene Pdcd1, located at 2q37.3, has a total length of 9.6 kb, consists of 5 exons and 4 introns, and contains a promoter of 663 bp at the upstream thereof. The PD1 is a type I transmembrane protein of 55 KDa. Its molecular structure consists of an extracellular region, a transmembrane region and an intracellular region. The extracellular region contains one immunoglobulin variable (IgV) domain, and the intracellular region contains an immunoreceptor tyrosin-based inhibitory motif (ITIM) and an immunoreceptor tyrosine-based switch motif (ITSM). The amino acid sequence of the extracellular region of the PD-1 has 24% homology with that of CTLA-4 and 28% homology with that of CD28. After T cells are activated, the PD-1 mainly gathers tyrosine phospholipases SHP2 through the ITIM, leading to dephosphorylation of downstream effector molecules.

The PD-1 has two ligands: PD-L1 and PD-L2. Both the PD-L1 and PD-L2 are B7 homologues. A PDL gene is located at the site 9P24.2 of a human chromosome, and has a length of 42 kb. Both the PD-L1 and PD-L2 have a molecular structure containing one immunoglobulin-like variable domain, one constant region-like domain, one transmembrane region, and one short cytoplasmic tail.

Upon binding with the PD-L1 and PD-L2, the PD-1 can down-regulate the activation of the T cells. The PD-L1 is expressed on the surfaces of a variety of tumor cells, including: lung cancer, gastric cancer, liver cancer, esophageal cancer, kidney cancer, ovarian cancer, cervical cancer, breast cancer, skin cancer, colon cancer, bladder cancer, glial cancer, head and neck cancer, and oral squamous cell carcinoma. Also, a large number of PD-L1-expressing CD8+ T cells are found in the periphery of these cancers. Statistics of clinical results show that the high expression level of the PD-L1 on tumor cells is related to the poor prognosis of a cancer patient.

### SUMMARY

The technical problem to be solved by the application is to overcome the defects and deficiencies of the existing antibodies with low binding affinity and specificity to PD-1, by providing an anti-PD-1 humanized antibody or an antigen-binding fragment thereof and an application thereof.

An objective of the application is to provide an anti-PD-1 humanized antibody or an antigen-binding fragment thereof. The antibody includes a light chain CDR region and a heavy chain CDR region. The heavy chain CDR region consists of HCDR1, HCDR2, and HCDR3. The light chain CDR region consists of LCDR1, LCDR2, and LCDR3. The amino acid sequences of the HCDR1, HCDR2, and HCDR3 are as shown in SEQ ID NOs:8-10 sequentially, and the amino acid sequences of the LCDR1, LCDR2, and LCDR3 are as shown in SEQ ID NOs:11-13 sequentially. The amino acid sequence of a heavy chain variable region of the antibody is as shown in any one of SEQ ID NOs:3-5.

The amino acid sequence of a light chain variable region of the antibody is as shown in any one of SEQ ID NOs:6-7.

The application further relates to a nucleic acid, vector, cell or pharmaceutical composition related to the antibody or the antigen-binding fragment thereof.

The application further relates to use of the antibody or the antigen-binding fragment thereof, and its related nucleic acid, vector, cell or pharmaceutical composition in the preparation of a medicament for treating a PD-1-mediated disease or condition.

The application further relates to a method for treating a PD-1-mediated disease or condition, the method including: allowing a subject to uptake an effective amount of the antibody or the antigen-binding fragment thereof, nucleic acid, vector, cell or pharmaceutical composition.

The application further relates to the antibody or the antigen-binding fragment thereof, nucleic acid, vector, cell or pharmaceutical composition for treating.

The application further relates to the antibody or the antigen-binding fragment thereof, nucleic acid, vector, cell or pharmaceutical composition for treating a PD-1-mediated disease or condition.

### Brief Description of the Drawings

Fig. 1 is a graph showing the effects of different concentrations of PD-1-112-C2 on the secretion of IL-2/IFN-γ.
Fig. 2 is a graph showing the effects of different concentrations of PD-1-112-C2 on the proliferation of T cells and the secretion of a cytokine IL-2 by T cells.
Fig. 3 is a graph showing the effects of different concentrations of PD-1-112-C2 on the proliferation of T cells and the secretion of a cytokine IFN-γ by T cells.
Fig. 4 is a graph showing the effect of an anti-PD-1 humanized antibody c53 on tumor volume.
Fig. 5 is a graph showing the effect of the anti-PD-1 humanized antibody c53 on the survival of mice.

### Detailed Description of the Embodiments

The present disclosure will be further explained below with reference to specific examples, but the examples do not limit the present disclosure in any form. Unless otherwise specified, the reagents, methods and devices used in the application are conventional reagents, methods and devices in the technical field.

Unless otherwise specified, the reagents and materials used in the following examples are commercially available.

The application relates to an anti-PD-1 humanized antibody or an antigen-binding fragment thereof. The antibody includes a light chain CDR region and a heavy chain CDR region. The heavy chain CDR region consists of HCDR1, HCDR2, and HCDR3. The light chain CDR region consists of LCDR1, LCDR2 and LCDR3. The amino acid sequences of HCDR1, HCDR2, and HCDR3 are as shown in SEQ ID NOs:8-10 sequentially, and the amino acid sequences of LCDR1, LCDR2, and LCDR3 are as shown in SEQ ID NOs:11-13 sequentially. The amino acid sequence of a heavy chain variable region of the antibody is as shown in any one of SEQ ID NOs:3-5. The amino acid sequence of a light chain variable region of the antibody is as shown in any one of SEQ ID NOs:6-7.

In the application, a CDR region is identified by employing a Kabat numbering system, but a CDR region identified by other methods also belongs to the claimed scope of the application.

In some implementations, the amino acid sequence of the heavy chain variable region of the antibody is as shown in SEQ ID NO:3 and the amino acid sequence of the light chain variable region of the antibody is as shown in SEQ ID NO:6, or the amino acid sequence of the heavy chain variable region of the antibody is as shown in SEQ ID NO:4 and the amino acid sequence of the light chain variable region of the antibody is as shown in SEQ ID NO:7, or the amino acid sequence of the heavy chain variable region of the antibody is as shown in SEQ ID NO:5 and the amino acid sequence of the light chain variable region of the antibody is as shown in SEQ ID NO:7.

In some implementations, the antibody contains a heavy chain constant region and a light chain constant region, the heavy chain constant region is any one or more of IgG1, IgG2, IgG3, IgG4, IgA, IgD, IgE or IgM; and the light chain constant region is a κ or λ chain.

In some implementations, a species source of the heavy chain constant region and the light chain constant region is selected from human, murine or monkey.

In some implementations, the antibody is a chimeric antibody or a polyspecific antibody (e.g., a bispecific antibody).

In the application, the term "polyspecific antibody" is an antigen-binding protein or antibody that targets more than one antigen or epitope.

In the application, the term "bispecific antibody" is a polyspecific antigen-binding protein or polyspecific antibody, and can be produced by various methods including, but not limited to fusion of hybridomas or linking of Fab' fragments. See, e.g., Songsivilai and Lachmann, 1990, Clin.Exp.Immunol.79:315-321; Kostelny et al., 1992, J.Immunol.148:1547-1553. The two binding sites of a bispecific antigen-binding protein or antibody will bind to two different epitopes present on the same or different protein targets.

In the application, the term "specific binding" or similar expressions thereof refers to the binding of an antibody or an antigen-binding fragment thereof to an epitope on a predetermined antigen. Typically, the antibody or the antigen-binding fragment thereof binds with affinity (K_{D}) of approximately less than 10⁻⁶ M, for example approximately less than 10⁻⁷ M, 10⁻⁸ M, 10⁻⁹ M or 10⁻¹⁰ M or less. The K_{D} refers to the ratio of a dissociation rate to a binding rate (koff/kon), which quantity can be measured by a method familiar to those skilled in the art.

In some implementations, the antigen-binding fragment is any one or more of F(ab')₂, Fab, scFv, Fv and a single domain antibody.

In the application, the term "F(ab')₂" includes two light chains and two heavy chains which contains part of a constant region between CH1 and CH2 domains so as to form an inter-chain disulfide bond between the two heavy chains. The F(ab')₂ fragment thus consists of two Fab' fragments held together by the disulfide bond between the two heavy chains.

In the application, the term "Fab" consists of one light chain, a CH1 and a variable region of one heavy chain. The heavy chain of the Fab molecule cannot form a disulfide bond with another heavy chain molecule.

In the application, the term "scFv" is an Fv molecule in which the variable regions of the heavy and light chains are linked by a flexible linker to form a single polypeptide chain (which forms the antigen-binding region) (see, e.g., Bird et al., Science.242:423-426 (1988) and Huston et al., Proc. Natl. Acad. Sci. USA.90:5879-5883 (1988)).

In the application, the term "Fv" includes variable regions from the heavy and light chains, but lacks a constant region.

In the application, the term "single domain antibody" contains only one heavy chain variable region (VHH) and two conventional CH2 and CH3 regions, but it is not as easy to stick to each other as an artificially engineered single chain antibody (scFv) and even aggregate into blocks. More importantly, the VHH structure cloned and expressed separately has comparable structural stability and antigen binding activity as the original heavy chain antibody, and is the smallest known unit that can bind to a target antigen.

The application further relates to a nucleic acid encoding the anti-PD-1 humanized antibody or the antigen-binding fragment thereof.

In a preferred implementation, the nucleic acid includes: a first nucleic acid encoding a heavy chain variable region of the antibody or the antigen-binding fragment thereof, and/or a second nucleic acid encoding a light chain variable region of the antibody or the antigen-binding fragment thereof.

In the application, the nucleic acid is usually an RNA or a DNA, and the nucleic acid molecule can be single-stranded or double-stranded, preferably a double-stranded DNA. The nucleic acid is "operably linked" when it is placed into a functional relationship with another nucleic acid sequence. For example, a promoter or enhancer is operably linked to a coding sequence if the promoter or enhancer affects the transcription of the coding sequence. DNA is preferably employed when the nucleic acid is ligated into a vector. Furthermore, since the antibody is a membrane protein, the nucleic acid usually carries a signal peptide sequence.

The application further relates to a vector carrying the nucleic acid.

In the application, the term "vector" is a nucleic acid vehicle into which a polynucleotide can be inserted. When a vector can enable a protein encoded by the inserted polynucleotide to be expressed, the vector is called an expression vector. The vector can be introduced into a host cell by transformation, transduction or transfection, so that a genetic material element carried by the vector can be expressed in the host cell. The vector is well known to those skilled in the art, including but not limited to: a plasmid; a phagemid; a cosmid: an artificial chromosome, for example a yeast artificial chromosome (YAC), a bacterial artificial chromosome (BAC) or a P1-derived artificial chromosome (PAC); a phage for example a λ or M13 phage, and an animal virus, etc. An animal virus that can be used as a vector includes, but is not limited to, a retrovirus (including a lentivirus), an adenovirus, an adeno-associated virus, a herpes virus (e.g. a herpes simplex virus), a poxvirus, a baculovirus, a papillomavirus, a papovavirus (e.g. SV40).

The application further relates to a cell carrying the nucleic acid, containing the vector or being capable of expressing the antibody or the antigen-binding fragment thereof.

The application further relates to a pharmaceutical composition including the antibody or the antigen-binding fragment thereof, the nucleic acid, the vector or the cell.

In the application, the term "pharmaceutical composition" is existed in a form that allows the biological activity of an active ingredient to be effective, and does not contain an additional ingredient that is unacceptably toxic to a subject to which the composition will be administered.

In some implementations, the pharmaceutical composition further includes a pharmaceutically acceptable carrier and/or excipient.

In the application, the term "pharmaceutically acceptable carrier" may include any and all solvents, dispersion media, coatings, antibacterial agents and antifungal agents, isotonic agents and absorption delaying agents, etc. that are physiologically compatible and used for extending the shelf life or potency of the antibody.

Moreover, the use of the antibody or the antigen-binding fragment thereof, the nucleic acid, the vector, the cell or the pharmaceutical composition in the preparation of a medicament for treating a PD-1-mediated disease or condition should also be within the claimed scope of the application.

In some implementations, the pharmaceutical composition or medicament is in a form suitable for injection.

In a preferred implementation, the pharmaceutical composition or medicament is in a form suitable for administration by subcutaneous injection, intradermal injection, intravenous injection, intramuscular injection or intralesional injection.

The application has the following beneficial effects.

The anti-PD-1 humanized antibody or the antigen-binding fragment thereof provided by the application can bind to a CHO-hPD1 cell, a CHO-cyno cell and an activated PBMC with high affinity. Its affinity is significantly improved compared with that of a positive control. It can bind to the PD-1 efficiently and specifically, and block the binding of the ligands PD-L1/PD-L2 to the CHO-hPD1 effectively. In MLR, it can block the binding of the PD-1 to the ligands and inhibit a PD-1 signaling pathway, thereby promoting the proliferation of T cells and the secretion of cytokines IL-2 and IFN-γ. Therefore, the antibody or the antigen-binding fragment thereof, and its related nucleic acid, vector, cell or pharmaceutical composition have broad application prospect in the preparation of a medicament for treating a PD-1-mediated disease or condition.

### Example 1 Preparation of anti-PD-1 antibody

### 1. Immunogen

A human PD-1 sequence (NCBI NP 005009) was artificially synthesized, and an upstream primer: 5'-CCGCAAGCTTGCCGCCACCATG-3' (SEQ ID NO:1), and a downstream primer: 5'-CCGGAATTCTCATTAATGGTGATGGTGATGATGCTGGAACTGGCCGGCAGGTC-3' (SEQ ID NO:2) were used for PCR amplification of extracellular terminal of the human PD-1 sequence. Then the extracellular terminal was double digested with Hind III and EcoRI and cloned into a pCDNA3.4A eukaryotic expression system, and formed a plasmid. A 293 cell was transfected with this plasmid, and the supernatant was harvested and then purified to obtain a human PD-1 recombinant protein (hPD-1).

### 2. Immunization of Animals

125 µg of the hPD-1 recombinant protein with a concentration of 1.23 mg/mL as an antigen was mixed with an equivalent amount of Freund's adjuvant (Sigma-Aldrich F5881) as an immune adjuvant, and five 6-week-old BAL b/C mice were taken and subjected to subcutaneous immunization, with the amount of immunized antigen per mouse being 25 µg. After the primary immunization, a booster immunization of the same dose was carried out once a week. After a total of 5 immunizations, the immune response was monitored by collecting tail blood of the mice. Fusion was performed by FACS screening (as described below) using mice with sufficient anti-hPD-1 immunoglobulin titers. 3 days after intraperitoneal booster immunization with the antigen, the mice were sacrificed and the spleen was taken out for cell fusion.

### 3. Selection of BAL b/C mice that produced the anti-hPD-1 antibody

To select for BAL b/C mice that produced the anti-hPD-1 antibody, the immunized mouse sera were tested by FACS. Serum dilutions from the mice immunized with the hPD-1 recombinant protein were incubated with hPD1-transfected CHO cells at 4°C for 30 minutes, washed with PBS for 3 times, then added with 0.4 µg/mL of PE goat anti-mouse IgG (Biolegend 405307) and incubated at 4°C for 30 minutes. After being washed with PBS for 3 times, the sample was put into a flow cytometer available from Beckman Coulter company (CytoFLEX A00-1-1102) for detection to validate whether it can bind to the hPD1-transfected CHO cells and screen out the BAL b/C mice that produce the anti-hPD-1 antibody, and then cell fusion was conducted.

### 4. Generation of hybridomas that produced mouse monoclonal antibodies against hPD-1

Splenocytes from the immunized BAL b/c mice were fused with mouse myeloma cells, and then the resulting hybridomas were screened for antigen-specific antibodies. A single cell suspension from the splenocytes of the immunized mice was subjected to cell fusion with one fifth of the mouse myeloma cells (SP2/0, ATCC CRL1581) which did not secrete an immunoglobulin, with PEG 1500 (Roche 10783641001). The fused cells were plated in a 96-well cell culture plate at about 1 × 10⁵ cells/well, and the cell culture plate was put into an incubator (Panasonic MCO-18AIC), and cultured under conditions of 37°C and 5% CO₂. It was subsequently cultured in a HAT selective medium which was a 1640 medium containing a 1X penicillin and streptomycin double antibody (Gibco 15140122), 1×HAT (Sigma CRLP-7185) and 20% fetal bovine serum (Royacel RY-F11-01) for about one week. After 1 week, the HAT medium was replaced with a HT medium (a 1640 medium containing a 1X penicillin and streptomycin double antibody (gibco 15140122), 1×HT (gibco 11067030) and 20% fetal bovine serum (Royacel RY-F11-01)) for culture, and then the cell culture supernatant of the fusion plate was detected by FACS to screen out hybridomas that secreted antibodies that could bind to the hPD-1 protein. The hybridomas that secreted antibodies that could bind to the hPD-1 protein were re-plated, and screened again. The hybridomas that were screened positive for the antibodies that bound to the hPD-1 protein, were subcloned at least twice by a limited dilution method. Stable subclones are then cultured in vitro and a small amount of antibodies are produced for further analysis. The hybridoma clone PD1-112-C2 was selected for further analysis.

### Example 2 Characterization of the affinity of anti-PD-1 mouse monoclonal antibody

According to a conventional method, a CHO (Chinese hamster ovary cell) cell line (CHO-hPD1) expressing a recombinant human PD-1 on a cell surface, a CHO cell line (CHO-cynoPD1) expressing a monkey PD1 (Uniprot: B0LAJ2) on a cell surface, and a CHO cell line (CHO-mousePD1) expressing a mouse PD1 (Uniprot: Q02242) on a cell surface were prepared by recombinant technology. The cell lines would be used for flow cytometry (FCM) to determine the binding characterization of the anti-PD-1 mouse monoclonal antibody PD-1-112C2.

In order to evaluate the binding of the anti-PD-1 mouse monoclonal antibody to the CHO-hPD1, 2 × 10⁵ CHO-hPD1 cells and an anti-PD-1 mouse monoclonal antibody diluted in concentration gradient (with an initial concentration of 10 µg/mL, and diluted in 3-fold gradient) were added into a 96-well plate, and incubated at 4°C for 30 minutes. The cells were washed once with a buffer (PBS containing 3% BSA) and then added with a PE-labeled anti-mouse IgG (Fc) Ab (Biolegend) fluorescent secondary antibody, incubated at 4°C for 30 minutes, then washed once with the buffer and subsequently resuspended in PBS. Thereafter, the cell suspension was subjected to flow cytometric analysis by CytoFlex (Beckman flow cytometer), and the quantity of the antibody bound to the cells was measured according to the mean fluorescence intensity (MFI) of the staining. The same method was used for evaluating the binding of the anti-PD-1 mouse monoclonal antibody to the CHO-cyno cells and the CHO-mousePD1 (sometimes abbreviated as "CHO-mPD1" in the application) cells.

The results were as shown in Table 1. The data indicated that each anti-PD-1 mouse monoclonal antibody PD-1-112-C2 could bind to both the CHO-hPD1 cells and the CHO-cyno cells with high affinity; and meanwhile, all mouse monoclonal antibody did not bind to the CHO-mousePD1 cells.

**Table 1**

| Antibody to be tested | FCM, EC₅₀, nM | | |
|---|---|---|---|
| | CHO-hPD1 | CHO-cynoPD1 | PBMC(activated) |
| PD-1-112-C2 | 0.739 | 0.463 | 0.109 |

### Example 3 Binding of anti-PD-1 antibody to activated PBMC

As stimulated by PHA (Sigma), among fresh human peripheral blood mononuclear cells (PBMCs), lymphocytes could be activated and proliferated, and reached the highest abundance in terms of expression of PD1 on the third day, and thus could be used for the experiment of binding of the PD-1 antibody to the PD1 naturally expressed by activated lymphocytes.

After being obtained from fresh human peripheral blood by gradient centrifugation of lymphatic separation medium, the PBMCs were adjusted to the density of 1 × 10⁶ cells/mL and inoculated into T75, and meanwhile added with PHA-L (Sigma) at a final concentration of 1 µg/mL to stimulate the proliferation of lymphocytes. After being allowed to stand in an incubator at 37°C and 5% CO₂ for 3 days, the cell suspension was taken out and centrifuged. The supernatant was discarded, and the precipitate was resuspended in a buffer (PBS containing 3% BSA) and added into a 96-well U-shaped plate at 2 × 10⁵/well, then added with a total of 10 concentration gradients of anti-PD1 antibodies diluted in 3-fold gradient starting from 30 µg/mL, incubated at 4°C for 30 minutes, and centrifuged at 300g for 5 minutes. The cells were washed once with the buffer, and added with a PE-labeled goat anti-human IgG fluorescent antibody (Biolegend), and incubated at 4°C for 30 minutes. The cells were centrifuged and washed once, then resuspended in PBS, and subsequently analyzed by a CytoFlex flow cytometer to detect the quantity of the antibody bound to the PBMC.

The results were as shown in Table 1, and the anti-PD1 antibody could bind to the activated lymphocytes with high affinity.

### Example 4 Binding specificity of anti-PD1 mouse monoclonal antibody

The anti-PD-1 mouse monoclonal antibody was allowed to bind with four different member proteins of CD28 family to verify the specificity of the antibody in binding to PD-1. Utilizing a standard ELISA method, PD-1, CD28, CTLA-4, and ICOS (ACRO) with a concentration of 1 µg/mL were immobilized on an ELISA plate, and added with the anti-human PD-1 mouse monoclonal antibody at a concentration of 10 µg/mL. A horse radish peroxidase (HRP)-coupled anti-mouse IgG (Sigma) was used as the secondary antibody. After developed with TMB and terminated, it was read for an OD450 value with a microplate reader.

The results were as shown in Table 2. Each anti-PD-1 mouse monoclonal antibody PD-1-112C2 could bind to the PD-1 specifically, but not to other members of CD28 family.

**Table 2**

| Antibody to be tested | human PD-1-hFc | human CD28-hFc | human ICOS-hFc | human CTLA4-hFc |
|---|---|---|---|---|
| PD-1-112-C2 | 2.1213 | 0.0625 | 0.062 | 0.131 |

### Example 5 Determination of affinity of anti-human PD-1 mouse monoclonal antibody by bio-layer interferometry (BLI) method

ForteBio (Octet Qke) affinity determination: a PD-1-his (ACRO) recombinant protein at a concentration of 5 µg/mL was loaded onto a HISIK biosensor for 120 seconds, then the loaded sensor was equilibrated in a standard buffer (PBST, PBS + 0.02% Tuween20) for 120 seconds, thereafter the sensor was transferred into a dilution of the anti-PD-1 mouse monoclonal antibody and stayed for 180 seconds to measure an association rate, and then transferred into the standard buffer for 20 minutes to measure a dissociation rate. Finally, analysis was conducted by utilizing a kinetic model.

The results of data processing were as shown in Table 3.

**Table 3**

| Antibody to be tested | Kon (1/Ms) | Kdis (1/s) | KD (M) |
|---|---|---|---|
| Opdivo(ABA0333) | 1.38E+06 | 3.63E-06 | 2.62E-12 |
| PD-1-112-C2 | 7.32E+05 | 3.18E-05 | 4.35E-11 |

### Example 6 Blocking the binding of ligand PD-L1/PD-L2 to CHO-hPD1 by anti-PD-1 mouse monoclonal antibody

The ability of the anti-PD-1 mouse monoclonal antibody to block the binding of the ligand to a PD-1 stably expressed on the surface of transfected CHO cells was analyzed by a flow cytometer. The ligand protein used in the experiment was a fusion protein of a recombinant PD-L1/PD-L2 extracellular segment linked to a human IgG Fc segment: PD-L1-hFc (ACRO) and PD-L2-hFc (ACRO).

The CHO-PD1 cells were resuspended with a buffer (PBS containing 3% BSA), and adjusted to the density of 2 × 10⁶ cells/mL. The cell suspension was added into a 96-well U-shaped plate at 100 µL/well and centrifuged at 300g for 5 minutes, and then the supernatant was discarded.

The follow-up process could be divided into two blocking modes: mode 1, in which the wells containing the cells were added with PD-L1-hFc/PD-L2-hFc at a concentration of 3 µg/mL, incubated at 4°C for 30 minutes, then added with a total of 10 concentration gradients of anti-PD-1 mouse monoclonal antibodies diluted in 3-fold gradient starting from 30 µg/mL, and incubated at 4°C for 30 minutes; and mode 2, in which the wells containing the cells were added with a total of 10 concentration gradients of anti-PD-1 mouse monoclonal antibodies diluted in 3-fold gradient starting from 30 µg/mL, incubated at 4°C for 30 minutes, then added with a PD-L1-hFc/PD-L2-hFc protein at a concentration of 3 µg/mL, and incubated at 4°C for 30 minutes.

The cells were centrifuged at 300g for 5 minutes, washed once with the buffer, added with a PE-labeled goat anti-human IgG fluorescent antibody (Biolegend), and incubated at 4°C for 30 minutes. The cells were centrifuged and washed once, then resuspended in PBS, and subsequently analyzed by a CytoFlex flow cytometer to detect the quantity of the ligand protein bound to the cells, and calculate the IC₅₀ value of the PD-1 antibody in blocking the binding.

The results were as shown in Table 4. The anti-PD-1 mouse monoclonal antibody PD-1-112-C2 could effectively block the binding of PD-L1/PD-L2 to CHO-PD1 cells in both modes.

**Table 4**

| Antibody to be tested | FCM, IC₅₀, nM | | | |
|---|---|---|---|---|
| | Blocking binding of CHO-hPD1 to PD-L1 | | Blocking binding of CHO-hPD1 to PD-L2 | |
| | mode 1 | mode 2 | mode 1 | mode 2 |
| PD-1-112-C2 | 2.567 | 1.546 | 4.614 | 3.727 |

### Example 7 Effect of anti-PD-1 antibody on cytokine release from SEB-stimulated PBMC cells

In this example, when overnight cultured peripheral blood mononuclear cells (PBMCs) were stimulated by the addition of Staphylococcus aureus enterotoxin B (SEB) as a superantigen, the effect on cytokine secretion was detected in the presence or absence of the anti-PD-1 antibody.

Fresh peripheral blood mononuclear cells (PBMCs) were resuspended with an X-VIVO 15 medium (LONZA) containing 10% FBS, then added into a T25 culture flask, and allowed to stand at 37°C and 5% CO₂ overnight for culture. On the next day, the suspended cells were taken, centrifuged, then resuspended in a fresh X-VIVO (containing 10% FBS) medium, and added with SEB as a superantigen (Toxin technology) at a final concentration of 200 ng/mL, then added into a 96-well plate at 1 × 10⁵ cells per well, and meanwhile added with different concentrations of anti-PD-1 antibodies. Additionally, two wells with isotype control antibodies (one is mlgG1 isotype control antibody (Biolegend); the other is hIgG4 isotype control antibody (Biolegend)), and a control well without antibody were set. After 3 days, a sample well was sampled, and measured for a IL-2/IFN-γ level with a IL2/IFN-γ Human Uncoated ELISA Kit (eBioscience).

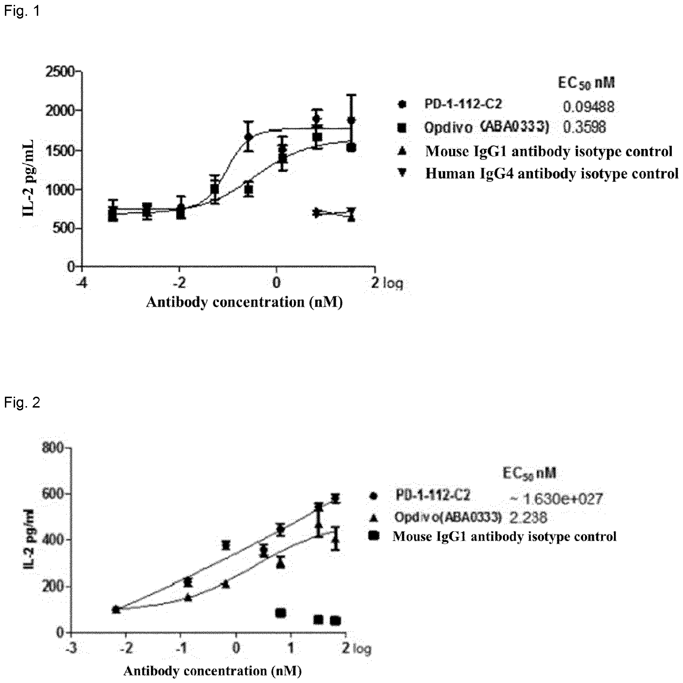

The result of the effect of different concentrations of PD-1-112-C2 on the secretion of IL-2/IFN-γ was as shown in Fig. 1. The anti-PD-1 antibody improves the secretion of IL-2/IFN-γ in a concentration-dependent manner. These results indicated that in the PBMCs stimulated by the SEB as the superantigen, anti-PD-1 antibody: PD-1-112-C2 could further promote the secretion of cytokines by T cells.

### Example 8 Effect of anti-PD-1 antibody in mixed lymphocyte reaction

In a mixed lymphocyte reaction (MLR), the presence or absence of the anti-PD-1 antibody could demonstrate proliferation of T cells and the level of cytokines secreted by the T cells in the situation that PD1 signaling was blocked.

CD14⁺ monocytes were isolated from fresh PBMCs with CD14 MicroBeads, human (Miltenyi), induced in the presence of GM-CSF/IL-4 for 6 days, then added with TNF-α, and induced for DC cells maturation after 3 days. On the day of the experiment, T cells in the PBMCs were purified with a EasySep^{™} Human T Cell Enrichment Kit (StemCell). 1 × 10⁵ of the T cells were mixed with 1×10⁴ of DC cells and cultured, and the mixed cells were added with different concentration gradients of anti-PD-1 antibodies. Additionally, two wells with isotype control antibodies (one is mlgG1 isotype control antibody and the other is hIgG4 isotype control antibody (Biolegend)), and a control well without the antibody were set. After 3 days of mixed culture, the supernatant was taken and detected for IL-2, and after another 2 days of culture, the supernatant was taken and detected for IFN-γ.

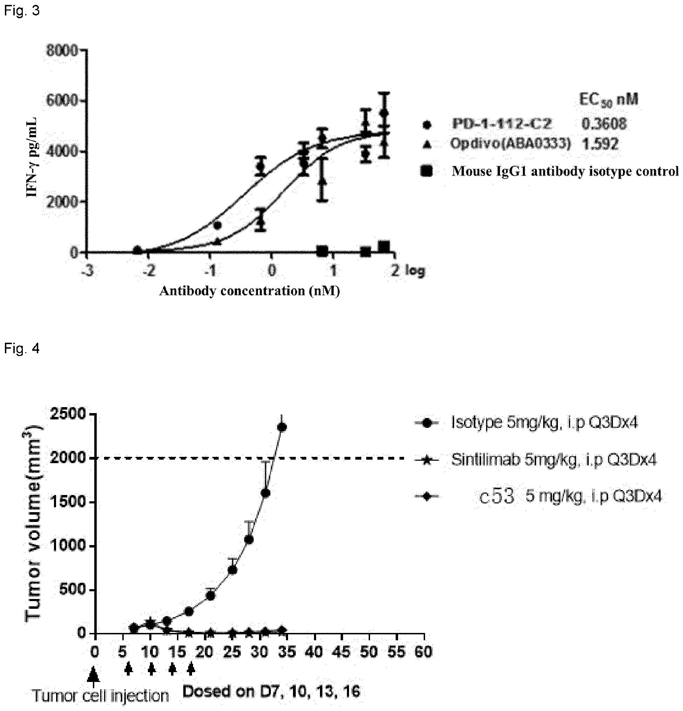

The results of the effects of different concentrations of PD-1-112-C2 on T cell proliferation and secretion of the cytokine IL-2 by the T cells were as shown in Fig. 2. The results of the effects of different concentrations of PD-1-112-C2 on T cell proliferation and secretion of the cytokine IFN-γ by the T cells were shown in Fig. 3. The results of Figs. 2 and 3 indicated that in the MLR experiment, the anti-PD-1 antibody: PD-1-112-C2 can block the binding of PD1 to the ligand in an antibody concentration-dependent manner and inhibits the PD1 signaling pathway, thereby promoting the proliferation of T cells and promoting the secretion of cytokines IL-2 and IFN-γ by the T cells.

### Example 9 Humanization of anti-PD-1 mouse monoclonal antibody

The anti-PD-1 mouse monoclonal antibody PD-1-112-C2 obtained above (the amino acid sequences of HCDR1, HCDR2, and HCDR3 of it were as shown in SEQ ID NOs: 8-10 sequentially, and the amino acid sequences of LCDR1, LCDR2, and LCDR3 of it were as shown in SEQ ID NOs:11-13 sequentially, the heavy chain variable region sequence of it was as shown in SEQ ID NO:14, and the light chain variable region sequence of it was as shown in SEQ ID NO:15) was humanized, and the specific method was as follows.

A human PD-1 sequence (NCBI NP 005009) was artificially synthesized and cloned into a pCDNA3.4A eukaryotic expression system. A 293 cell was transfected with this plasmid, and the supernatant was harvested and then purified to obtain a human PD-1 recombinant protein. Female BAL b/C mice were immunized with the obtained human PD-1 recombinant protein subcutaneously. The splenocytes from the immunized BAL b/C mice were fused with mouse myeloma cells, and then the resulting hybridomas were screened for antigen-specific antibodies. The hybridomas that were screened positive for the antibodies that bound to the hPD-1 protein, were subcloned at least twice by a limited dilution method, and then the stable subclones were cultured in vitro to generate a small amount of antibodies which were further screened to obtain PD-1-112-C2 clones.
SEQ ID NO:8: TYYMY.
SEQ ID NO:9: GINPSNGGTNFNEKFKS.
SEQ ID NO:10: RDSNYDGGFDY
SEQ ID NO:11: RASKSVSTSGYSYMH.
SEQ ID NO:12: LAYHLES.
SEQ ID NO:13: QHSWELPIT.
SEQ ID NO:14: 
QVQLQQPGAELVKPGASVKLSCKASGYTFTTYYMYWVKQRPGQGLEWIGGINPSNGGTNFN
EKFKSKATLTVDKSSSTAYMQLSSLTSEDSAVYYCTRRDSNYDGGFDYWGQGTTLTVSS.
SEQ ID NO:15:

DIVLTQSPASLAVSLGQRATISCRASKSVSTSGYSYMHWYQQKPGQPPKLLIYLAYHLESGVP
ARFSGSGSGTDFTLNIHPVEEEDAATYYCQHSWELPITFGSGTKLEIKR.

Referring to SEQ ID NO:14 and SEQ ID NO:15, a humanized template that best matches its non-CDR region was selected from a Germline database. The template of the heavy chain of the antibody was IGHV1, and the template of the light chain of the antibody was IGKV1. Under the principle of enhancing structural stability without affecting the structural stability of the antibody, without affecting the binding of the antibody to the antigen, without introducing protein modification sites such as glycosylation and phosphorylation, and without introducing sites such as sites that easily been oxidized or aminated, the humanized heavy chain sequence was designed as VH1-5, and the humanized light chain sequence was designed as VL1-3, and meanwhile, for the light chain, taking IGKV7-3*01 with the highest homology as another set of humanized design templates, a humanized sequence VL-4 was designed. The pairing form of the light and heavy chains was designed to be IGHV1/IGKV2 as a common pairing form, so as to obtain humanized antibodies.

Genes were synthesized according to the amino acid sequence of each light and heavy chain of the humanized antibodies, and subjected to double enzyme digestion with Hind III (NEB) and EcoRI (NEB). Then the gene fragments were inserted onto a pDNA3.4A expression vector (Invitrogen) through the Hind III (NEB) /EcoRI (NEB) enzyme cleavage sites by employing T4 DNA ligase (TAKARA 2011A). HEK293 cells (Life Technologies Cat.NO.11625019) were transfected with the expression vector and a transfection reagent PEI (Poly science, Inc. Cat. NO.23966) at a ratio of 1:2, and placed and cultured in a CO₂ incubator for 5-7 days. The expressed antibody was recovered by centrifugation, and then purified according to a conventional method to obtain the anti-PD-1 humanized antibodies (c11, c21, c31, c41, c51, c12, c22, c32, c42, c52, c43, c53, c44, c54) of the application; wherein, the amino acid sequences of 3 strains of anti-PD-1 humanized antibodies (c22, c43, c53) were as shown in Table 5.

**Table 5 Amino acid sequences of 3 strains of anti-PD-1 humanized antibodies (c22, c43, c53)**

| Anti-PD-1 humanized antibody | Heavy chain variable region (VH) | Light chain variable region (VL) |
|---|---|---|
| c22 | SEQ ID NO:3 | SEQ ID NO:6 |
| c43 | SEQ ID NO:4 | SEQ ID NO:7 |
| c53 | SEQ ID NO:5 | SEQ ID NO:7 |

SEQ ID NO:3: 
QVQLVQSGAEVKKPGASVKVSCKASGYTFTTYYMYWVRQAPGQGLEWMGGINPSNGGTNF
NEKFKSRVTMTVDKSTSTAYMELSSLRSEDTAVYYCTRRDSNYDGGFDYWGQGTTVTVSS.
SEQ ID NO:4: 
QVQLVQSGAEVKKPGASVKVSCKASGYTFTTYYMYWVRQAPGQGLEWIGGINPSNGGTNYA
EKFKGRVTLTVDTSTSTAYMELSSLRSEDTAVYYCTRRDSNYDGGFDYWGQGTTVTVSS.
SEQ ID NO:5: 
QVQLVQSGAEVKKPGASVKVSCKASGYTFTTYYMYWVRQAPGQGLEWMGGINPSNGGTNY
AQKFQGRATMTVDTSTSTAYMELSSLRSEDTAVYYCTRRDSNYDGGFDYWGQGTTVTVSS.
SEQ ID NO:6: 
DIQLTQSPSSLSASVGDRATITCRASKSVSTSGYSYMHWYQQKPGKAPKLLIYLAYHLESGVP
SRFSGSGSGTDFTLTISSVQPEDFATYYCQHSWELPITFGQGTKLEIKR.
SEQ ID NO:7:

DIQMTQSPSSLSASVGDRVTITCRASKSVSTSGYSYMHWYQQKPGKAPKLLIYLAYHLESGV
PSRFSGSGSGTDFTLTISSLQPEDFATYYCQHSWELPITFGQGTKLEIKR.

### Example 10 Affinity Characterization of anti-PD-1 humanized antibody

### 1. Experimental method

A CHO (Chinese Hamster Ovary Cells) cell line, CHO-hPD1, capable of expressing a recombinant human PD-1 on the cell surface, and a CHO cell line, CHO-cynoPD1, capable of expressing a monkey PD1 on the cell surfacewere prepared by recombinant technology. The two cell lines would be used for flow cytometry (FCM) to determine the binding characterization of the candidate humanized anti-PD-1 monoclonal antibodies (c22, c43, c53). The specific method was:
In order to evaluate the binding of the humanized antibody to the CHO-hPD1, 2 × 10⁵ CHO-hPD1 cells and the humanized antibody diluted in concentration gradient (with an initial concentration of 30 µg/mL, and diluted in 3-fold gradient) were added into a 96-well plate, and incubated at 4°C for 30 minutes. The cells were washed once with a buffer (PBS containing 3% BSA) and then added with a PE-labeled anti-human IgG (Fc) Ab (Biolegend) fluorescent secondary antibody, incubated at 4°C for 30 minutes, then washed once with the buffer and subsequently resuspended in PBS. Thereafter, the cell suspension was subjected to flow cytometric analysis by CytoFlex (Beckman flow cytometer), and the quantity of the antibody bound to the cells was measured according to the mean fluorescence intensity (MFI) of the staining. The same method was used for evaluating the binding of the humanized antibody to the CHO-cyno cells.

### 2. Experimental results

The affinity characterization of the anti-PD-1 humanized antibody was shown in Table 6, and the results indicated that the anti-PD-1 humanized antibody of the application can bind to both the CHO-hPD1 cells and the CHO-cynoPD1 cells with high affinity.

**Table 6 Affinity characterization of anti-PD-1 humanized antibody**

| Serial number | Degree of humanization | CHO-hPD1 | CHO-cynoPD1 | Activated PBMC |
|---|---|---|---|---|
| | | FCM, EC50, nM | | |
| c11 | 96.35% | 1.715 | 0.7967 | 0.1927 |
| c21 | 97.40% | 1.725 | 0.9276 | 0.2235 |
| c31 | 97.40% | 2.45 | 0.9835 | 0.2131 |
| c41 | 97.92% | 2.107 | 0.7721 | 0.2216 |
| c51 | 99.22% | 1.687 | 0.8502 | 0.1886 |
| C12 | 95.83% | 2.119 | 0.8698 | 0.1724 |
| c22 | 96.88% | 1.677 | 0.6299 | 0.1411 |
| c32 | 96.88% | 2.214 | 0.697 | 0.2761 |
| c42 | 97.40% | 1.441 | 0.66 | 0.253 |
| c52 | 98.70% | 1.591 | 0.7943 | 0.2977 |
| c43 | 98.18% | 1.58 | 0.8945 | 0.2815 |
| c53 | 99.48% | 1.71 | 0.7427 | 0.213 |
| c44 | 96.35% | 1.651 | 0.7091 | 0.2119 |
| c54 | 97.66% | 2.183 | 0.8956 | 0.2402 |

### Example 11 Binding of anti-PD-1 humanized antibody to activated PBMC

### 1. Experimental method

As stimulated by PHA (Sigma), among fresh human peripheral blood mononuclear cells (PBMCs), lymphocytes could be activated and proliferated, and reached the highest abundance in terms of expression of PD1 on the third day, and thus could be used for the experiment of binding of the anti-PD-1 humanized antibody (c22, c43, c53) to the PD1 naturally expressed by activated lymphocytes. The specific method was:
After being obtained from fresh human peripheral blood by gradient centrifugation of lymphatic separation medium, the PBMCs were adjusted to the density of 1 × 10⁶ cells/mL and inoculated into T75, and meanwhile added with PHA-L (Sigma) at a final concentration of 1 µg/mL to stimulate the proliferation of lymphocytes. After being allowed to stand in an incubator at 37°C and 5% CO₂ for 3 days, the cell suspension was taken out and centrifuged. The supernatant was discarded, and the precipitate was resuspended in a buffer (PBS containing 3% BSA) and added into a 96-well U-shaped plate at 2E5/well, then added with different concentration gradients of the humanized antibody, incubated at 4°C for 30 minutes, and then centrifuged at 300g for 5 minutes. The cells were washed once with the buffer, and added with a PE-labeled goat anti-human IgG fluorescent antibody (Biolegend), and incubated at 4°C for 30 minutes. Then cells were centrifuged and washed once, resuspended in PBS, and subsequently analyzed by a CytoFlex flow cytometer to detect the quantity of the antibody bound to the PBMC.

### 2. Experimental results

The results of the determination of the binding ability of the anti-PD-1 humanized antibody to activated PBMCs were as shown in Table 6, and the results indicated that the anti-PD-1 humanized antibody of the application could bind to the activated lymphocytes with high affinity.

Compared with the anti-PD-1 mouse monoclonal antibodies PD1-112-C2 (Table 1) in Examples 2 and 3, the anti-PD-1 humanized antibodies (c22, c43, c53) of the application had comparable capacity of binding to the CHO-hPD1 cells, the CHO-cyno cells and the activated PBMCs.

### Example 12 Binding specificity of anti-PD1 humanized antibody

### 1. Experimental method

The anti-PD1 humanized antibodies (c22, c43, c53) of the application were allowed to bind with four different member proteins of CD28 family to verify the specificity of the anti-PD1 humanized antibody in binding to PD-1. Utilizing a standard ELISA method, PD-1 (Aero), CD28 (Aero), CTLA-4 (Aero), and ICOS (Aero) with a concentration of 1 µg/mL were respectively immobilized on an ELISA plate, and added with the humanized antibody at a concentration of 10 µg/mL. A horse radish peroxidase (HRP)-coupled anti-human IgG (F) was used as the secondary antibody. After developed with TMB and terminated, it was read for an OD450 value with a microplate reader.

### 2. Experimental results

The results of the binding specificity of the anti-PD-1 humanized antibody were as shown in Table 7, and the results indicated that the anti-PD-1 humanized antibody of the application could specifically bind to PD-1, but not to other members of CD28 family.

**Table 7 Binding specificity results of anti-PD-1 humanized antibody**

| Antibody to be tested | human PD-1-hFc | human CD28-hFc | human ICOS-hFc | human CTLA4-hFc |
|---|---|---|---|---|
| c22 | 1.3256 | 0.1799 | 0.1170 | 0.2090 |
| c43 | 1.2296 | 0.1890 | 0.1311 | 0.2549 |
| c53 | 1.4767 | 0.1761 | 0.1327 | 0.2388 |

### Example 13 Affinity determination of anti-PD1 humanized antibody

### 1. Experimental method

ForteBio (Octet Qke) affinity determination: a PD-1-his recombinant protein at a concentration of 5 µg/mL was loaded onto a HISIK biosensor for 120 seconds, then the loaded sensor was equilibrated in a standard buffer (PBST, PBS + 0.02% Tuween20) for 120 seconds, thereafter the sensor was transferred into a dilution of the anti-PD-1 humanized antibody (c22, c43, c53) and stayed for 180 seconds to measure the association rate, and then transferred into the standard buffer for 20 minutes to measure the dissociation rate. Finally, analysis was conducted by utilizing a kinetic model, and data processing was conducted. Opdivo (ABA0333) was used as a positive control.

### 2. Experimental results

The results of the determination of the affinity of the anti-PD-1 humanized antibody was as shown in Table 8, and the results indicated that all the anti -PD-1 humanized antibodies of the application could bind to PD-1 with high affinity.

Compared with the anti-PD-1 mouse monoclonal antibody PD-1-112-C2 (Table 3) in Example 5, the anti-PD-1 humanized antibody of the application has comparable affinity; and compared with the positive control Opdivo (Table 3), the affinity of the anti-PD-1 humanized antibody of the application was significantly improved.

**Table 8 Affinity determination results of anti-PD-1 humanized antibody**

| Antibody to be tested | Kon (1/Ms) | Kdis (1/s) | KD (M) |
|---|---|---|---|
| c22 | 9.85E+05 | < 1.0E-07 | C1.0E-12 |
| c43 | 9.84E+05 | < 1.0E-07 | C1.0E-12 |
| c53 | 9.79E+05 | < 1.0E-07 | C1.0E-12 |

### Example 14 Blocking the binding of ligand PD-L1/PD-L2 to CHO-hPD1 by anti-PD-1 humanized antibody

### 1. Experimental method

The ability of the anti-PD-1 humanized antibody to block the binding of the ligand to a transfected CHO cells which could stably express PD-1 on the surface was analyzed by a flow cytometer. The ligand protein used in the experiment was a fusion protein of a recombinant PD-L1/PD-L2 extracellular segment linked to a mouse IgG1 Fc segment: PD-L1-mFc and PD-L2-mFc. The ligand proteins in the experiment were two recombinant fusion proteins: PD-L1-mFc and PD-L2-mFc, which are formed by connecting the extracellular segments of PD-L1/PD-L2 with the mouse IgG1 Fc segment.

The CHO-PD1 cells were resuspended with a buffer (PBS containing 3% BSA), and adjusted to the density of 2 × 10⁶ cells/mL. The cell suspension was added into a 96-well U-shaped plate at 100 µL/well and centrifuged at 300g for 5 minutes, and then the supernatant was discarded. The well containing the cells was added with PD-L1-mFc/PD-L2-mFc at a concentration of 0.2 µg/mL, incubated at 4°C for 30 minutes, then added with the anti-PD-1 humanized antibodies (c22, c43, c53) diluted in concentration gradient, and incubated at 4°C for 30 minutes.

The cells were centrifuged at 300g for 5 minutes, washed once with the buffer, added with a PE-labeled goat anti-mouse IgG fluorescent antibody (Biolegend), and incubated at 4°C for 30 minutes. The cells were centrifuged and washed once, then resuspended in PBS, and subsequently analyzed by a CytoFlex flow cytometer to detect the quantity of the ligand protein bound to the cells, and calculate the IC₅₀ value of the PD-1 antibody in blocking the binding.

### 2. Experimental results

The results of the determination of the affinity of the anti-PD-1 humanized antibody were as shown in Table 9, and the results indicated that all anti-PD-1 humanized antibodies of the application could effectively block the binding of PD-L1/PD-L2 to the cells CHO-PD1.

**Table 9 Affinity determination results of anti-PD-1 humanized antibody**

| Antibody to be tested | FCM, IC₅₀, nM | |
|---|---|---|
| | Blocking binding of CHO-hPD1 to PD-L1 | Blocking binding of CHO-hPD1 to PD-L2 |
| c22 | 3.100 | 6.876 |
| c43 | 3.143 | 7.951 |
| c53 | 3.024 | 7.049 |

### Example 15 Effect of anti-PD1 humanized antibody on mixed lymphocyte reaction

### 1. Experimental method

In a mixed lymphocyte reaction (MLR), the presence or absence of the anti-PD-1 humanized antibody could demonstrate proliferation of T cells and the level of cytokines secreted by the T cells in the situation that PD1 signaling was blocked. The specific method was:
CD14⁺ monocytes were isolated from fresh PBMCs with CD14 MicroBeads, human (Miltenyi), induced in the presence of GM-CSF/IL-4 for 6 days, then added with TNF-α, and induced for DC cells maturation after 3 days. On the day of the experiment, T cells in the PBMCs were purified with a EasySep^{™} Human T Cell Enrichment Kit (StemCell). 1×10⁵ of the T cells per well were mixed with 1×10⁴ of DC cells per well and cultured, and the mixed cells were added with different concentration gradients of anti-PD-1 humanized antibodies (c22, c43, c53). Additionally, a well with an isotype control antibody and a control well without the antibody were set. After 3 days of mixed culture, the supernatant was taken and detected for IL-2, and after another 2 days of culture, the supernatant was taken and detected for IFN-γ.

### 3. Experimental results

The results of the effect of the anti-PD-1 humanized antibody in the mixed lymphocyte reaction was as shown in Table 10. The results indicated that in the MLR experiment, the anti-PD-1 humanized antibody of the application could block the binding of the PD1 to the ligand and inhibit the PD1 signaling pathway, thereby promoting the proliferation of T cells and promoting the secretion of cytokines IL-2 and IFN-γ by the T cells.

**Table 10 Effect of anti-PD-1 humanized antibody in mixed lymphocyte reaction**

| Antibody to be tested | IL2 EC50 | IFNγ EC50 |
|---|---|---|
| | nM | nM |
| c22 | 13.34 | 0.7274 |
| c43 | 2.792 | 2.735 |
| c53 | 1.881 | 6.649 |

Example 16 Evaluation of *In vivo* anti-tumor efficacy of anti-PD-1 humanized antibody on mouse colon cancer cells

### 1. Experimental method

Purpose of the experiment: to determine the *in vivo* anti-tumor activity of the anti-PD-1 humanized antibody (c53) against mouse colon cancer cells (MC38 cells), an isotype control group (Isotype) and a positive control group (Sintilimab) were set simultaneously.

Experimental materials: 6-8 weeks old female hPD1 Knock in mice (C57BU6 background, source: Beijing Vitalstar Biotechnology Co., Ltd.): MC38 cells (National Infrastructure of Cell Line Resource); FBS (Gibco, 10091-148), 0.25% trypsin-EDTA (Gibco, 25200056), DMSO (Sigma, D2650), DPBS (Hyclone, SH30028.02), penicillin-streptomycin (Gibco, 15140122), a DMEM high glucose medium (Gibco, 11965084), fetal bovine serum (Gibco), and glutamine (Gibco).

Instruments and equipment: an electronic balance (Shanghai Sunny Hengping Scientific Instrument Co., Ltd., JA12002), a vernier caliper (Shanghai Menet Industrial Co., Ltd., MNT-150T), a microscope (Chongqing Optec Instrument Co., Ltd., BDS200), a medical centrifuge (Hunan Xiangyi Laboratory Instrument Development Co., Ltd., L530R), a digital display constant temperature water bath (Precision Machinery Co.,Ltd., HH-S), a carbon dioxide incubator (Panasonic Healthcare Co., Ltd., Japan, MCO-18AC), a double vertical type ultra-clean bench (Wuxi Yichun Purification Equipment Co., Ltd., SW-CJ-VS2).

### Experimental steps:

Cell culture: MC38 cells were cultured in a DMEM high glucose medium containing 10% fetal bovine serum, 1% glutamine and 1% penicillin-streptomycin (1:1).

Inoculation: MC38 cells in the logarithmic growth phase were collected, and adjusted to the cell concentration of 3 × 10⁶ cells/mL. 40 female hPD1 mice were taken and subcutaneously inoculated with the MC38 cells with an inoculation volume of 0.1 mL/mouse, i.e., 3 ×10⁵ cells/mouse.

Administration: the day of inoculation was recorded as day 0 (D0). On day 7, the mice were randomly divided into 3 groups according to the tumor volume, with 8 mice in each group, and the administration was started (the administration dosage, manner and frequency of the MC38 tumor model were as shown in Table 11).

**Table 11**

| Groups | Times and mode of administration | Administration volume | Number of animals per group |
|---|---|---|---|
| Isotype (5 mg/kg) | D7, D10, D13, D16 Intraperitoneal (IP) | 0.2mL-10gbw⁻¹ | 8 |
| c53 (5 mg/kg) | | | |
| Sintilimab (5 mg/kg) | | | |

Recording: the measurement and recording of the tumor volume was started on D7, and then the long and short diameters of the tumor were measured with a vernier caliper twice a week. The tumor volume was calculated with the formula: (1/2) × long diameter × (short diameter)². When each mouse reached the end point of the experiment (the tumor volume exceeded 2000 mm³ reached a humane endpoint), the mice were sacrificed by cervical dislocation, and the survival curve was recorded.

### 2. Experimental results

The results of the effect of the anti-PD-1 humanized antibody on the tumor volume were as shown in Table 12 and Fig. 4. It could be seen that compared with the Isotype group, the anti-PD-1 humanized antibody (c53) had a significant inhibitory effect on the tumor growth of the MC38 tumor model (TGI = 104.44%, 7 mice with tumor complete elimination), which was comparable to the anti-tumor effect of the Sintilimab group (TGI = 105.81%, 7 mice were completely free of tumor).

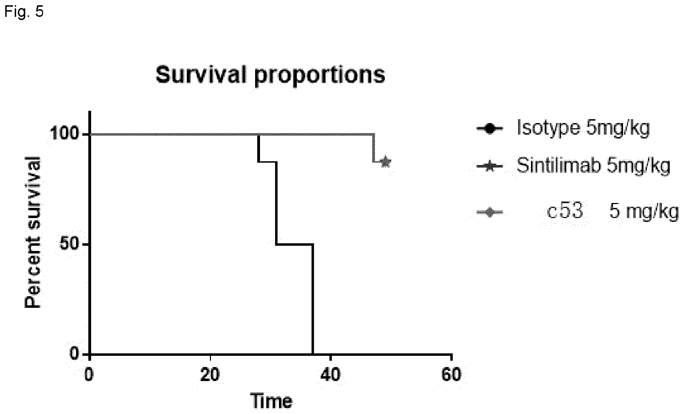

The results of the effect of the anti-PD-1 humanized antibody on the survival of the mice were as shown in Fig. 5. It could be seen that compared with the Isotype group, the anti-PD-1 humanized antibody (c53) could obviously prolong the survival of the mice.

**Table 12 Effect of anti-PD-1 humanized antibody on tumor volume (mm³)**

| Days after administration | Isotype (5 mg/kg) | c53 (5mg/kg) | Sintilimab (5 mg/kg) |
|---|---|---|---|
| 7 | 67. 54 | 64.80 | 66.28 |
| 10 | 103.33 | 106.47 | 136.76 |
| 13 | 149.58 | 41.79 | 41.96 |
| 17 | 256.60 | 15.31 | 12.02 |
| 21 | 437.02 | 10.52 | 6.80 |
| 25 | 728.07 | 14.17 | 5.40 |
| 28 | 1078.98 | 19.88 | 7.49 |
| 31 | 1011.44 | 28.18 | 18.07 |
| 34 | 2356.39 | 46.12 | 26.61 |

The above results indicated that the anti-PD-1 humanized antibody (c53) provided by the application could significantly inhibit the growth of MC38 cells, effectively prolong the survival of the mice, and had a significant curative effect on treating colon cancer in the mice.

The above embodiments are preferred embodiments of the application. However, the implementation of the application is not limited by the above embodiments. Any other change, modification, substitution, combination, and simplification made without departing from the spiritual essence and principle of the application should be an equivalent replacement manner, and all are included in a claimed scope of the application.

## Claims

1. An anti-PD-1 humanized antibody or an antigen-binding fragment thereof, comprising a light chain CDR region and a heavy chain CDR region, wherein the heavy chain CDR region consists of HCDR1, HCDR2, and HCDR3, and the light chain CDR region consists of LCDR1, LCDR2, and LCDR3, the amino acid sequences of the HCDR1, HCDR2, and HCDR3 are as shown in SEQ ID NOs:8-10 sequentially, and the amino acid sequences of the LCDR1, LCDR2, and LCDR3 are as shown in SEQ ID NOs:11-13 sequentially, wherein the amino acid sequence of a heavy chain variable region of the antibody is as shown in any one of SEQ ID NOs:3-5.

2. The antibody or the antigen-binding fragment thereof according to claim 1, wherein the amino acid sequence of a light chain variable region of the antibody is as shown in any one of SEQ ID NOs:6-7.

3. The antibody or the antigen-binding fragment thereof according to claim 2, wherein the amino acid sequence of the heavy chain variable region of the antibody is as shown in SEQ ID NO:3 and the amino acid sequence of the light chain variable region of the antibody is as shown in SEQ ID NO:6, or
the amino acid sequence of the heavy chain variable region of the antibody is as shown in SEQ ID NO:4 and the amino acid sequence of the light chain variable region of the antibody is as shown in SEQ ID NO:7, or
the amino acid sequence of the heavy chain variable region of the antibody is as shown in SEQ ID NO:5 and the amino acid sequence of the light chain variable region of the antibody is as shown in SEQ ID NO:7.

4. The antibody or the antigen-binding fragment thereof according to any one of claims 1-3, wherein the antibody contains a heavy chain constant region and a light chain constant region, the heavy chain constant region is any one or more of IgG1, IgG2, IgG3, IgG4, IgA, IgD, IgE or IgM; and the light chain constant region is a κ or λ chain.

5. The antibody or the antigen-binding fragment thereof according to any one of claims 1-4, wherein the antibody is a chimeric antibody or a polyspecific antibody; and the antigen-binding fragment is any one or more of F(ab')₂, Fab, scFv, Fv and a single domain antibody.

6. A nucleic acid encoding the anti-PD-1 humanized antibody or the antigen-binding fragment thereof according to any one of claims 1-5;
wherein preferably, the nucleic acid comprises: a first nucleic acid encoding a heavy chain variable region of the antibody or an antigen-binding fragment thereof, and/or a second nucleic acid encoding a light chain variable region of the antibody or an antigen-binding fragment thereof.

7. A vector carrying the nucleic acid according to claim 6.

8. A cell carrying the nucleic acid according to claim 6, comprising the vector according to claim 7, or being capable of expressing the antibody or the antigen-binding fragment thereof according to any one of claims 1-5.

9. A pharmaceutical composition comprising the antibody or the antigen-binding fragment thereof according to any one of claims 1-5, the nucleic acid according to claim 6, the vector according to claim 7, or the cell according to claim 8.

10. Use of the antibody or the antigen-binding fragment thereof according to any one of claims 1-5, the nucleic acid according to claim 6, the vector according to claim 7, the cell according to claim 8, or the pharmaceutical composition according to claim 9 in the preparation of a medicament for treating a PD-1-mediated disease or condition.

11. A method for treating a PD-1-mediated disease or condition, comprising:
administering an effective amount of the antibody or the antigen-binding fragment thereof according to any one of claims 1-5, the nucleic acid according to claim 6, the vector according to claim 7, the cell according to claim 8 or the pharmaceutical composition according to claim 9.

12. The antibody or the antigen-binding fragment thereof according to any one of claims 1-5, the nucleic acid according to claim 6, the vector according to claim 7, the cell according to claim 8, or the pharmaceutical composition according to claim 9 for use as a medicament.

13. The antibody or the antigen-binding fragment thereof according to any one of claims 1-5, the nucleic acid according to claim 6, the vector according to claim 7, the cell according to claim 8, or the pharmaceutical composition according to claim 9 for use in a method for treating a PD-1-mediated disease or condition.
